# EUROPÄISCHE PATENTSCHRIFT

(11) **EP 0 602 097 B1**
(45) Veröffentlichungstag und Bekanntmachung des Hinweises auf die Patenterteilung: **24.04.1996**
(21) Anmeldenummer: 92918311.9
(22) Anmeldetag: 02.09.1992
(51) Int. Cl.: C07D 513/14, C07D 498/14, A61K 31/42, A61K 31/425

(54) **TRICYCLISCHE THIAZOL- UND OXAZOL-DERIVATE MIT ANTIVIRALER WIRKUNG**
TRICYCLIC THIAZOL AND OXAZOL DERIVATES WITH ANTIVIRAL ACTION
DERIVES TRICYCLIQUES DE THIAZOLE ET D'OXAZOLE A ACTION ANTIVIRALE

(30) Priorität: 07.09.1991 DE 4129779
(43) Veröffentlichungstag der Anmeldung: 22.06.1994
(73) Patentinhaber: BOEHRINGER MANNHEIM GMBH, D-68298 Mannheim (DE)
(72) Erfinder: ZILCH, Harald, D-6800 Mannheim 31 (DE); LEINERT, Herbert, D-6148 Heppenheim (DE); MERTENS, Alfred, D-6905 Schriesheim (DE)
(74) Vertreter: Mink, Reinhold, Dr.
(86) Internationale Anmeldenummer: EP9202015
(87) Internationale Veröffentlichungsnummer: WO9305047

(56) Entgegenhaltungen:
- WO-A-92/08457
- WO-A-92/09606
- WO-A-92/13863
- GB-A- 1 039 117
- US-A- 3 336 306
- JOURNAL OF HETEROCYCLIC CHEMISTRY. Bd. 26, Nr. 5, September 1989, PROVO US Seiten 1441 - 1445 M. K. AHMED ET AL. 'The reaction of arylmagnesium bromides with N-(.omega.-bromoalkyl)phthalimides.'

## Beschreibung

Gegenstand der vorliegenden Erfindung sind tricyclische Thiazol- und Oxazol-Derivate, Verfahren zu deren Herstellung und Arzneimittel, die diese Verbindungen enthalten.

Die Erfindung betrifft tricyclische Thiazolo-[2,3-a]pyrrol- und Oxazolo-[1,2-a]pyrrol-Derivate der Formel I 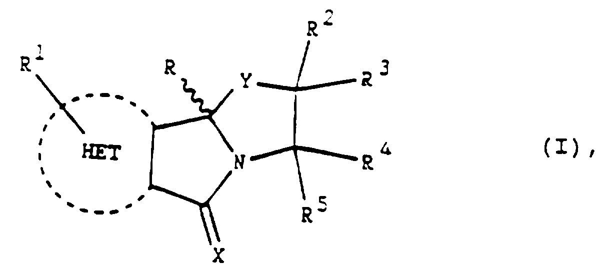 in der
- HET: einen aromatischen, teilhydrierten oder hydrierten hetero-cyclischen Ring mit 3-7 Ringatomen darstellt, von denen bis zu vier Atome Heteroatome sein können, wobei die Heteroatome gleich oder verschieden sein können und Sauerstoff, Stickstoff oder Schwefel bedeuten, und die Heterocyclen gegebenenfalls an einem oder mehreren Stickstoffatomen ein Sauerstoffatom tragen können, wobei HET durch einen, zwei oder drei Reste R¹, die gleich oder verschieden sein können, substituiert sein kann,
- Y: ein Sauerstoff- oder Schwefelatom, bzw. die SO oder SO₂- Gruppe darstellt,
- X: ein Sauerstoff- oder Schwefelatom sein kann,
- R: einen geradkettigen oder verzweigten, gesättigten oder ungesättigten aliphatischen Rest mit 1-9 C-Atomen, der durch Phenyl substituiert sein kann, oder
einen Phenylring oder einen mono-, bi- oder tricyclischen carbocyclischen Ring mit 7-15 C-Atomen oder ein heterocyclisches mono-, bi- oder tricyclisches Ringsystem mit jeweils fünf oder sechs Ringatomen bedeutet und pro Ringsystem 1-4 bzw. 1-5 Heteroatome enthalten sein können und die Heteroatome Stickstoff, Schwefel oder Sauerstoff sind, wobei die Heteroatome Stickstoff und Schwefel gegebenenfalls durch Sauerstoffatome substituiert sein können,
und die vorgenannten Phenylringe, die mono-, bi- oder tricyclischen carbocyclischen Ringe oder das heterocyclische mono-, bi- oder tricyclische Ringsystem gegebenenfalls ein- oder mehrfach substituiert ist durch C₁-C₆-Alkyl, C₁-C₆-A-koxy, C₁-C₆-Alkylmercapto, C₁-C₆-Alkylsulfinyl, C₁-C₆-Alkylsulfonyl, C₂-C₆-Alkenyl, C₂-C₆-Alkinyl, C₂-C₆-Alkenyloxy, C₂-C₆-Alkenylmercapto, C₂-C₆-Alkinyloxy, C₂-C₆-Alkinylmercapto, Amino, C₁-C₆-Alkylamino, Di-C₁-C₆-alkylamino, C₁-C₆-Alkylcarbonylamino, C₁-C₆-Alkylamino-carbonyl, C₁-C₆-Alkoxycarbonyl, Hydroxy, Benzyloxy, Phenylmercapto, Phenyloxy, Nitro, Cyano, Halogen, Trifluormethyl, Azido, Formylamino, Carboxy oder Phenyl,
- R¹: ein Wasserstoffatom, einen geradkettigen oder verzweigten, gesättigten oder ungesättigten aliphatischen Rest mit 1-6 C-Atomen oder C₁-C₆-Alkoxy, C₁-C₆-Alkylmercapto, C₁-C₆-Alkylsulfinyl, C₁-C₆-Alkylsulfonyl, Amino, C₁-C₆-Alkylamino, Di-C₁-C₆-Alkylamino, Sulfonamido, C₁-C₆-Alkoxycarbonyl, Carboxy, Halogen, Hydroxy, Nitro, Cyano, Azido, Phenyl oder Benzyloxy bedeutet,
- R: Wasserstoff, C₁-C₆-Alkyl, C₁-C₆-Alkoxy, C₁-C₆-Alkylmercapto, Amino, C₁-C₆-Alkylamino, Di-C₁-C₆-Alkylamino, Halogen, Cyano, Hydroxy, Carboxy, C₁-C₆-Alkoxycarbonyl, Aminocarbonyl oder C₁-C₆-Alkylaminocarbonyl bedeutet,
- R³, R⁴, R⁵: die gleiche Bedeutung wie R haben, wobei die Reste R, R³, R⁴ und R⁵ unabhängig voneinander gleich oder verschieden sein können, und R³ mit R⁴ gegebenenfalls eine Doppelbindung darstellen kann,
sowie deren Tautomere, Enantiomere, Diastereomere und physiologisch verträgliche Salze.

Thiazolo-[2,3-a]isoindole sind aus der früheren deutschen Patentanmeldung P 40 35 809.7 (WO 92/08457) als antivirale Arzneimittel bekannt.

Der vorliegenden Erfindung lag die Aufgabe zugrunde, neue tricylische Thiazolo-[2,3-a]pyrrole und Oxazolo-[1,2-a]pyrrole mit pharmakologischen Eigenschaften zur Verfügung zu stellen. Diese Aufgabe wird durch die in den Ansprüchen gekennzeichneten Merkmale gelöst.

Die Verbindungen der vorliegenden Erfindung weisen wertvolle pharmakologische Eigenschaften auf. Insbesondere besitzen sie eine antivirale Wirksamkeit und eignen sich zur Therapie und Prophylaxe von Infektionen, die durch DNA-Viren wie z.B. das Herpes-Simplex-Virus, das Zytomegalie-Virus, Papilloma-Viren, das Varicella-Zoster-Virus oder Epstein-Barr-Virus oder RNA-Viren wie Toga-Viren oder insbesondere Retroviren wie die Onko-Viren HTLV-I und II, sowie die Lentiviren Visna und Humanes-Immunschwäche-Virus HIV-1 und -2, verursacht werden.

Besonders geeignet erscheinen die Verbindungen der Formel I zur Behandlung der klinischen Manifestationen der retroviralen HIV-Infektion beim Menschen, wie der anhaltenden, generalisierten Lymphadenopathie (PGL), dem fortgeschrittenen Stadium des AIDS-verwandten Komplex (ARC) und dem klinischen Vollbild von AIDS.

Die erfindungsgemäßen Verbindungen der allgemeinen Formel I besitzen eine ausgeprägte antivirale Wirkung und eignen sich insbesondere zur Behandlung von viralen bzw. retroviralen Infektionen. Virale Infektionen von Säugern, insbesondere des Menschen, sind weit verbreitet. Trotz intensiver Bemühungen ist es bisher nicht gelungen, Chemotherapeutika bereitzustellen, die ursächlich oder symptomatisch mit dem viral oder retroviral bedingten Krankheitsgeschehen mit erkennbar substantiellem Erfolg interferieren. Es ist heutzutage nicht möglich, bestimmte Viruserkrankungen, wie zum Beispiel das Acquired Immune Deficiency Syndrom (AIDS), den AIDS-related-complex (ARC) und deren Vorstadien, Herpes-, Cytomegalie-Virus (CMV)-, Influenza- und andere Virusinfektionen zu heilen oder chemotherapeutisch deren Symptome günstig zu beeinflussen. Derzeit steht beispielsweise für die Behandlung von AIDS fast ausschließlich das 3'-Azido-3'-deoxy-thymidin (AZT), bekannt als Zidovudine oder Retrovir^{R}, zur Verfügung. AZT ist jedoch durch eine sehr enge therapeutische Breite bzw. durch bereits im therapeutischen Bereich auftretende, sehr schwere Toxizitäten charakterisiert (Hirsch, M.S. (1988) J.Infec.Dis. 157, 427-431). Die Verbindungen der allgemeinen Formel I besitzen diese Nachteile nicht. Sie wirken antiviral, ohne in pharmakologisch relevanten Dosen cytotoxisch zu sein.

Es konnte nun nachgewiesen werden, daß Verbindungen der allgemeinen Formel I die Vermehrung von DNA- bzw. RNA-Viren auf der Stufe der virusspezifischen DNA- bzw. RNA-Transkription hemmen. Die Substanzen können über die Inhibierung des Enzyms Reverse Transkriptase die Vermehrung von Retroviren beeinflussen (vgl. Proc. Natl. Acad. Sci. USA 83, 1911, 1986 bzw. Nature 325, 773 1987).

Da ein sehr großer Bedarf an Chemotherapeutica besteht, die möglichst spezifisch mit retroviral bedingten Erkrankungen oder deren Symptomen interferieren, ohne die normal ablaufenden natürlichen Körperfunktionen zu beeinflussen, könnten die genannten Verbindungen vorteilhaft prophylaktisch oder therapeutisch bei der Behandlung von Krankheiten eingesetzt werden, bei denen eine retrovirale Infektion von pathophysiologischer, symptomatischer oder klinischer Relevanz ist.

Die Trennung der Racemate in die Enantiomeren kann analytisch, semipräparativ und präparativ chromatographisch auf geeigneten optisch aktiven Phasen mit gängigen Elutionsmitteln durchgeführt werden.

Als optisch aktive Phasen eignen sich beispielsweise optisch aktive Polyacrylamide oder Polymethacrylamide, z.T. auch an Kieselgel (z.B. ChiraSpher ^{(R)} von Merck, Chiralpak ^{(R)} OT/OP von Baker), Celluloseester/-carbamate (z.B. Chiracel ^{(R)} OB/OY von Baker/Daicel), Phasen auf Cyclodextrin- oder Kronenetherbasis (z.B. Crownpak ^{(R)} von Daicel) oder mikrokristallines Cellulosetriacetat (Merck).

Der annelierte aromatische, teilhydrierte oder hydrierte heterocyclische Ring HET besitzt 3-7 Kohlenstoffatome, wobei 1-4 dieser Ringatome durch die Heteroatome Sauerstoff, Schwefel und/oder Stickstoff ersetzt sein können. Beispielhaft seien die folgenden Heterocyclen genannt: der Aziridin-, Furan-, Tetrahydrofuran-, Thiophen-, Thiolan-, Sulfonal-, Pyrrol-, Pyrrolidin-, Oxazol-, Oxazolin-, Isoxazol-, Thiazol-, Thiazolidin-, Pyrazol-, Pyrazolin-, Imidazol-, Imidazolin-, Oxadiazol-, Furan-, Triazol-, Pyridin-, Piperidin-, Morpholin-, Thiazin-, Pyridazin-, Pyrimidin-, Pyrazin-, Piperazin- oder Azepinring, sowie deren N-Oxide.

Im Fall der teilhydrierten Ringe können vorzugsweise ein oder zwei Doppelbindungen des aromatischen Systems hydriert sein, so daß die entsprechenden Di- oder Tetrahydroderivate resultieren. HET kann vorzugsweise durch einen oder zwei Reste R¹ substituiert sein, die unabhängig voneinander gleich oder verschieden sein können.

Ein aliphatischer Rest in der Definition von R und R¹ bedeutet einen geradkettigen oder verzweigten Alkyl-, Alkenyl- oder Alkinylrest mit 1-9, vorzugsweise 2-7 Kohlenstoffatomen, wie z.B. der Propyl-, Isopropyl-, Butyl-, Isobutyl-, Pentyl-, Hexyl- oder Heptylrest. Als ungesättigte Reste kommen C₂-C₇-Alkenyl- und Alkinylreste in Frage, bevorzugt C₂-C₅, wie z.B. der Allyl-, Dimethylallyl-, Butenyl-, Isobutenyl-, Pentenyl- oder Propinylrest.

Ein aliphatischer Rest, der durch Phenyl substituiert sein kann, ist insbesondere eine Phenyl-C₁-C₆-alkylgruppe, wie z.B. der Benzyl-, Phenethyl-, Phenylpropyl- oder Phenylbutylrest.

Die in der Definition von R genannten Phenylringe können ein-, zwei- oder dreifach substituiert sein. Die Substituenten können unabhängig voneinander in o-, m- oder p-Stellung stehen.

Ein carbocyclischer Ring mit 7-15 C-Atomen kann mono-, bi- oder tricyclisch sein und pro Ring jeweils 5 oder 6 C-Atome aufweisen. Dieser Ring kann gesättigt, ungesättigt, teilweise gesättigt oder aromatisch sein. Beispielhaft genannt seien die folgenden Ringsysteme: der Naphthyl-, Anthracenyl-, Phenanthrenyl-, Flourenyl-, Indenyl-, Acenaphthylenyl-, Norbornyl-, Adamantylring oder eine C₃-C₇-Cycloalkyl- oder C₅-C₈-Cycloalkenylgruppe. Der carbocyclische Ring kann darüberhinaus mono- oder disubstituiert sein, wobei die Substituenten unabhängig voneinander bevorzugt in o- oder m-Stellung stehen können.

Die heterocylischen mono-, bi- oder tricyclischen Ringsysteme des Restes R enthalten pro Ring 5 oder 6 Kohlenstoffatome, wobei 1-4 bzw. 1-5 C-Atome durch die Heteroatome Sauerstoff, Schwefel und/oder Stickstoff ersetzt sein können. Die Ringsysteme können aromatisch, partiell oder vollständig hydriert sein. Beispielhaft genannt seien die folgenden Ringsysteme: das Pyridin-, Pyrimidin-, Pyridazin-, Pyrazin- , Triazin-, Pyrrol-, Pyrazol-, Imidazol-, Triazol-, Thiazol-, Oxazol-, Isoxazol-, Oxadiazol-, Furazan-, Furan- , Thiophen-, Indol-, Chinolin-, Isochinolin-, Cumaron-, Thionaphthen-, Benzoxazol-, Benzthiazol-, Indazol-, Benzimidazol-, Benztriazol-, Chromen-, Phthalazin-, Chinazolin-, Chinoxalin-, Methylendioxybenzol-, Carbazol-, Acridin-, Phenoxazin-, Phenothiazin-, Phenazin- oder Purinsystem, sowie deren N-Oxide, wobei die ungesättigten bzw. aromatischen Carbo- und Heterocyclen partiell oder vollständig hydriert sein können. Das heterocyclische Ringsystem kann darüberhinaus mono- oder disubstituiert sein, wobei die Substituenten unabhängig voneinander bevorzugt in o- oder m-Stellung stehen können.

R bedeutet bevorzugt unsubstituiertes Phenyl oder Phenyl ein-oder zweifach substituiert durch C₁-C₃-Alkyl, C₁-C₃-Alkoxy, C₁-C₃-Alkylmercapto, C₁-C₃-Alkylsulfinyl, C₁-C₃-Alkylsulfonyl, C₂-C₄-Alkenyl, C₂-C₃-Alkinyl, C₃-C₄-Alkenyloxy, C₁-C₃-Alkylamino, C₁-C₃-Dialkylamino-, C₁-C₃-Alkylcarbonylamino, C₁-C₃-Alkylaminocarbonyl, C₁-C₃-Alkoxycarbonyl-, Amino, Hydroxy, Nitro, Azido, Trifluormethyl, Cyano oder Halogen.

Carbocyclische Ringe sind bevorzugt Biphenyl, Naphthyl, Anthracenyl, Indenyl, Fluorenyl, Acenaphthylenyl, Phenanthrenyl, Norbornyl, Adamantyl, C₃-C₆-Cycloalkyl, C₅-C₈-Cycloalkenyl, wobei die carbocyclischen Ringe ein- oder zweifach substituiert sein können durch C₁-C₃-Alkyl, C₁-C₃-Alkoxy, C₁-C₃-Alkylmercapto, C₁-C₃-Alkylsulfinyl, C₁-C₃-Alkylsulfonyl, C₂-C₄-Alkenyl, C₂-C₃-Alkinyl, C₃-C₄-Alkenyloxy, C₁-C₃-Alkylamino, C₁-C₃-Dialkylamino-, C₁-C₃-Alkylcarbonylamino, C₁-C₃-Alkylaminocarbonyl, C₁-C₃-Alkoxycarbonyl-, Amino, Hydroxy, Nitro, Azido, Trifluormethyl, Cyano oder Halogen.

Heterocyclische Ringsysteme des Restes R sind bevorzugt Pyrrol, Imidazol, Furan, Thiophen, Pyridin, Pyrimidin, Thiazol, Triazin, Indol, Chinolin, Isochinolin, Cumaron, Thionaphthen, Benzimidazol, Chinazolin, Methylendioxybenzol, Ethylendioxybenzol, Carbazol, Acridin und Phenothiazin, wobei die hetero-cyclischen Ringe ein-oder zweifach substituiert sein können durch C₁-C₃-Alkyl, C₁-C₃-Alkoxy, C₁-C₃-Alkylmercapto, C₁-C₃-Alkylsulfinyl, C₁-C₃-Alkylsulfonyl, C₂-C₄- Alkenyl, C₂-C₃-Alkinyl, C₃-C₄-Alkenyloxy, C₁-C₃-Alkylamino, C₁-C₃-Dialkylamino-, C₁-C₃-Alkylcarbonylamino, C₁-C₃-Alkylaminocarbonyl, C₁-C₃-Alkoxycarbonyl-, Amino, Hydroxy, Nitro, Azido, Trifluormethyl, Cyano oder Halogen.

Für den Rest R¹ ist Wasserstoff, C₁-C₃-Alkyl, C₂-C₄-Alkenyl, C₂-C₄-Alkinyl, C₁-C₃-Alkoxy, C₁-C₃-Alkylmercapto, C₁-C₃-Alkylamino, C₁-C₃-Alkoxycarbonyl, Amino, Halogen, Hydroxy, Cyano und Azido bevorzugt.

Bevorzugte Substituenten für R, R³, R⁴ und R⁵ sind Wasserstoff, C₁-C₃-Alkyl, C₁-C₃-Alkoxy, C₁-C₃-Alkylmercapto, Carboxy, C₁-C₃-Alkoxycarbonyl, Aminocarbonyl, C₁-C₆-Alkylaminocarbonyl, Halogen, Cyano und Hydroxy sowie die aus R³ und R⁴ gemeinsam gebildete Doppelbindung im Ring.

X ist bevorzugt Sauerstoff, Y bevorzugt gleich Sauerstoff und Schwefel. Unter Halogen ist allgemein Fluor, Chlor, Brom und Iod zu verstehen, bevorzugt Fluor, Chlor und Brom.

Bevorzugte annelierte Heterocyclen HET sind aromatische, teilhydrierte oder hydrierte stickstoffhaltige Ringe mit 3-7 Ringatomen, besonders bevorzugt mit 5 oder 6 Ringatomen.

Besonders bevorzugte Reste für R sind C₃-C₅-Alkyl, C₂-C₅-Alkenyl, C₂-C₄-Alkinyl, Benzyl, Phenethyl, Phenyl, durch C₁-C₃-Alkyl, C₁-C₃-Alkoxy, C₁-C₃-Alkylmercapto, Allyl, Allyloxy, C₁-C₃-Alkylamino, Di-C₁-C₃-alkylamino, Amino, Hydroxy, Azido, Trifluormethyl, Cyano oder Halogen mono- oder disubstituiertes Phenyl bzw. durch Methyl oder Halogen trisubstituiertes Phenyl, Naphthyl, Anthracenyl, Indenyl, Acenaphthylenyl, Phenanthrenyl, Adamantyl, Cyclohexyl, Cyclohexenyl, Furyl, Thienyl, Pyridyl, Pyrimidinyl, Thiazolyl, Indolyl, Chinolinyl, Benzimidazolyl, Methylendioxyphenyl, Carbazolyl und Phenothiazinyl und durch Methyl oder Halogen mono- oder disubstituierte Derivate der vorgenannten carbocyclischen oder heterocyclischen Ringe.

Für R¹ ist besonders bevorzugt Wasserstoff, Methyl, Ethyl, Isopropyl, Allyl, Methoxy, Ethoxy, Methylmercapto, Ethylmercapto, Methylamino, Methoxycarbonyl, Ethoxycarbonyl, Amino, Azido, Cyano, Hydroxy und Halogen, wobei Chlor und Brom für Halogen ganz besonders bevorzugt sind.

Für R, R³, R⁴ und R⁵ sind Methyl, Ethyl, Isopropyl, Methoxy, Ethoxy, Methylmercapto, Ethylmercapto, Methylamino, Amino, Chlor, Brom und Cyano besonders bevorzugt.

Insbesondere bevorzugt für HET sind der Pyrrol-, Oxazol-, Isoxazol-, Thiazol-, Imidazol-, Pyridin-, Piperidin-, Pyridazin-, Pyrimidin- und Pyrazinring.

Insbesondere bevorzugt sind Verbindungen der Formel I, in denen R, R¹, X und n die oben angegebene Bedeutung haben und R, R³, R⁴ und R⁵ gleich Wasserstoff, Methyl, Ethyl, Chlor, Brom, Methoxy oder Ethoxy sind, wobei R bis R⁵ besonders bevorzugt Wasserstoff darstellen, bzw. R bis R⁴ gleich Wasserstoff sind und R⁵ C₁-C₄-Alkoxycarbonyl, Aminocarbonyl oder C₁-C₃-Alkylaminocarbonyl darstellt.

Insbesondere bevorzugt sind Verbindungen der Formel I, in denen R³ und R⁴ gemeinsam eine Doppelbindung darstellen, R ein Wasserstoffatom ist und R⁵ die oben angegebene Bedeutung hat.

Die Arzneimittel enthalten mindestens eine Verbindung der Formel I zur Behandlung von viralen Infektionen können in flüssiger oder fester Form enteral oder parenteral appliziert werden. Hierbei kommen die üblichen Applikationsformen in Frage, wie beispielsweise Tabletten, Kapseln, Dragees, Sirupe, Lösungen oder Suspensionen. Als Injektionsmedium kommt vorzugsweise Wasser zur Anwendung, das die bei Injektionslösungen üblichen Zusätze wie Stabilisierungsmittel, Lösungsvermittler und Puffer enthält. Derartige Zusätze sind z.B. Tartrat- und Zitratpuffer, Ethanol, Komplexbildner, wie Ethylen-diamintetraessigsäure und deren nichttoxischen Salze, hochmolekulare Polymere, wie flüssiges Polyethylenoxid zur Viskositätsregulierung. Flüssige Trägerstoffe für Injektionslösungen müssen steril sein und werden vorzugsweise in Ampullen abgefüllt. Feste Trägerstoffe sind beispielsweise Stärke, Lactose, Mannit, Methylcellulose, Talkum, hochdisperse Kieselsäuren, höher molekulare Fettsäuren, wie Stearinsäure, Gelatine, Agar-Agar, Calziumphosphat, Magnesiumstearat, tierische und pflanzliche Fette, feste hochmolekulare Polymere, wie Polyethylenglykole, etc.. Für orale Applikationen geeignete Zubereitungen können gewünschtenfalls Geschmacks- oder Süßstoffe enthalten.

Die Dosierung kann von verschiedenen Faktoren, wie Applikationsweise, Spezies, Alter oder individuellem Zustand abhängen. Die erfindungsgemäßen Verbindungen werden üblicherweise in Mengen von 0,1 - 100 mg, vorzugsweise 0,2 - 80 mg pro Tag und pro kg Körpergewicht appliziert. Bevorzugt ist es, die Tagesdosis auf 2-5 Applikationen zu verteilen, wobei bei jeder Applikation 1-2 Tabletten mit einem Wirkstoffgehalt von 0,5 - 500 mg verabreicht werden. Die Tabletten können auch retardiert sein, wodurch sich die Anzahl der Applikationen pro Tag auf 1-3 vermindert. Der Wirkstoffgehalt der retardierten Tabletten kann 2 - 1000 mg betragen. Der Wirkstoff kann auch durch Dauerinfusion gegeben werden, wobei die Mengen von 5 - 1000 mg pro Tag normalerweise ausreichen.

Die Verbindungen der vorliegenden Erfindung und ihre pharmazeutische Zubereitungen können auch in Kombination mit anderen Arzneimitteln zur Behandlung und Prophylaxe der oben genannten Infektionen eingesetzt werden. Beispiele dieser weiteren Arzneimittel beinhalten Mittel, die zur Behandlung und Prophylaxe von HIV-Infektionen oder diese Krankheit begleitende Erkrankungen einsetzbar sind, wie z.B. 3'-Azido-3'-desoxythymidin; 2',3'-Didesoxynucleoside, wie z.B. 2',3'-Didesoxycytidin, 2',3'-Didesoxyadenosin oder 2',3'-Didesoxyinosin; acyclische Nucleoside (z.B. Acyclovir); Interferone, wie z.B. A-Interferon; renale Ausscheidungsinhibitoren, wie z.B. Probenicid; Nucleosid-Transport-Inhibitoren, wie z.B. Dipyridamol; Immunmodulatoren, wie z.B. Interleukin II oder Stimulierungsfaktoren, wie z.B. Granulocyten-Makrophagen-Kolonie-Faktor. Die Verbindungen der vorliegenden Erfindung und die anderen Arzneimittel können jeweils einzeln, gleichzeitig gegebenenfalls in einer einzigen oder zwei getrennten Formulierungen oder zu unterschiedlichen Zeiten verabreicht werden, so daß ein synergistischer Effekt erreicht wird.

Die erfindungsgemäßen Verbindungen der allgemeinen Formel I werden nach literaturbekannten Verfahren hergestellt, indem man heterocyclische Ketocarbonsäuren der allgemeinen Formel II 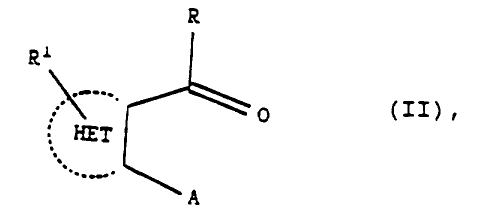 in der R und R¹ die oben angegebene Bedeutung haben und A gleich -COOH ist, mit substituiertem oder unsubstituiertem Cysteamin bzw. Ethanolamin der allgemeinen Formel III 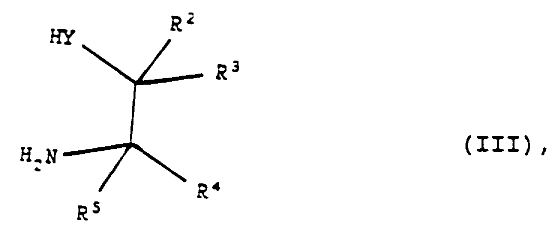 in der Y gleich Sauerstoff oder Schwefel ist und R, R³, R⁴ und R⁵ die oben angegebene Bedeutung haben, in einem geeigneten inerten Lösungsmittel bei Raumtemperatur bis Rückflußtemperatur evtl. in Gegenwart katalytischer Mengen Säure, z.B. p-Toluolsulfonsäure, umsetzt und gegebenenfalls anschließend Verbindungen der Formel I in andere Verbindungen der Formel I nachträglich umwandelt und anschließend chromatographisch bzw. durch Umkristallisation reinigt. Racemate können durch Chromatographie an geeigneten optisch aktiven Phasen, z.B. Cellulosetriacetat, in die Antipoden getrennt werden.

Die nachträglichen Umwandlungen von Verbindungen der Formel I in andere Verbindungen der Formel I betreffen z.B. die Herstellung von tricyclischen Thiazolo-[2,3-a]pyrrol-Derivaten mit X=S. Verbindungen mit X=S werden hergestellt durch Umsetzung von Verbindungen der Formel I, in der X ein Sauerstoffatom bedeutet, mit schwefelgruppenübertragenden Verbindungen, wie z.B. Lawesson's Reagenz.

Die Ketocarbonsäurederivate der allgemeinen Formel II sind z.T. literaturbekannt und werden z.B. durch Friedel-Crafts-Acylierung von substituierten oder unsubstituierten Anhydriden heterocyclischer Dicarbonsäuren der Formel IV 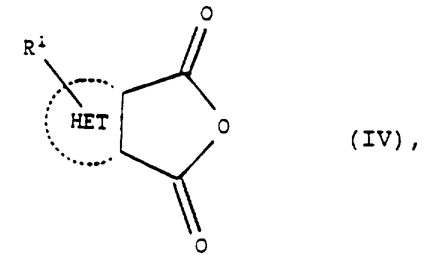 in der R¹ und Het die oben angegebene Bedeutungen haben, mit gegebenenfalls substituierten Arenen in Gegenwart einer Lewis-Säure (z.B. Aluminiumchlorid) oder durch Reaktion von Grignardreagenzien der allgemeinen Formel V

R-MgBr (V),

oder lithiumorganischen Verbindungen der allgemeinen Formel VI

R-Li (VI),

in denen R mit Ausnahme von Wasserstoff die oben angegebene Bedeutung hat, mit entsprechenden Anhydriden, die gegebenenfalls substituiert sind, in geeigneten inerten Lösungsmitteln bei tiefen Temperaturen hergestellt.

Die Verbindungen der Formel III sind literaturbekannt oder können analog zu den bekannten Verfahren hergestellt werden. Einige Derivate sind auch käuflich zu erwerben.

Die Verfahren zur Herstellung der erfindungsgemäßen Verbindungen der allgemeinen Formel I erfolgen im übrigen anaolg zu den in den folgenden Anmeldungen oder Literaturstellen beschriebenen Verfahren: US-Patent 3,334,113, CH-469,733, belgische Patentanmeldung 659,528 bzw. US-Patent 3,646,022, U.S. 2,860,985, belgische Patentanmeldung 564,592, J. Org. Chem. 30, 1506 (1965) sowie J. Org. Chem. 34, 165 (1969).

Im Sinne der vorliegenden Erfindung kommen außer den in den Beispielen genannten Verbindungen und der durch Kombination aller in den Ansprüchen genannten Bedeutungen der Substituenten die folgenden Verbindungen der Formel I in Frage, die als racemischen Gemische oder in optisch aktiver Form bzw. als reine R- und S-Enantiomere vorliegen können:

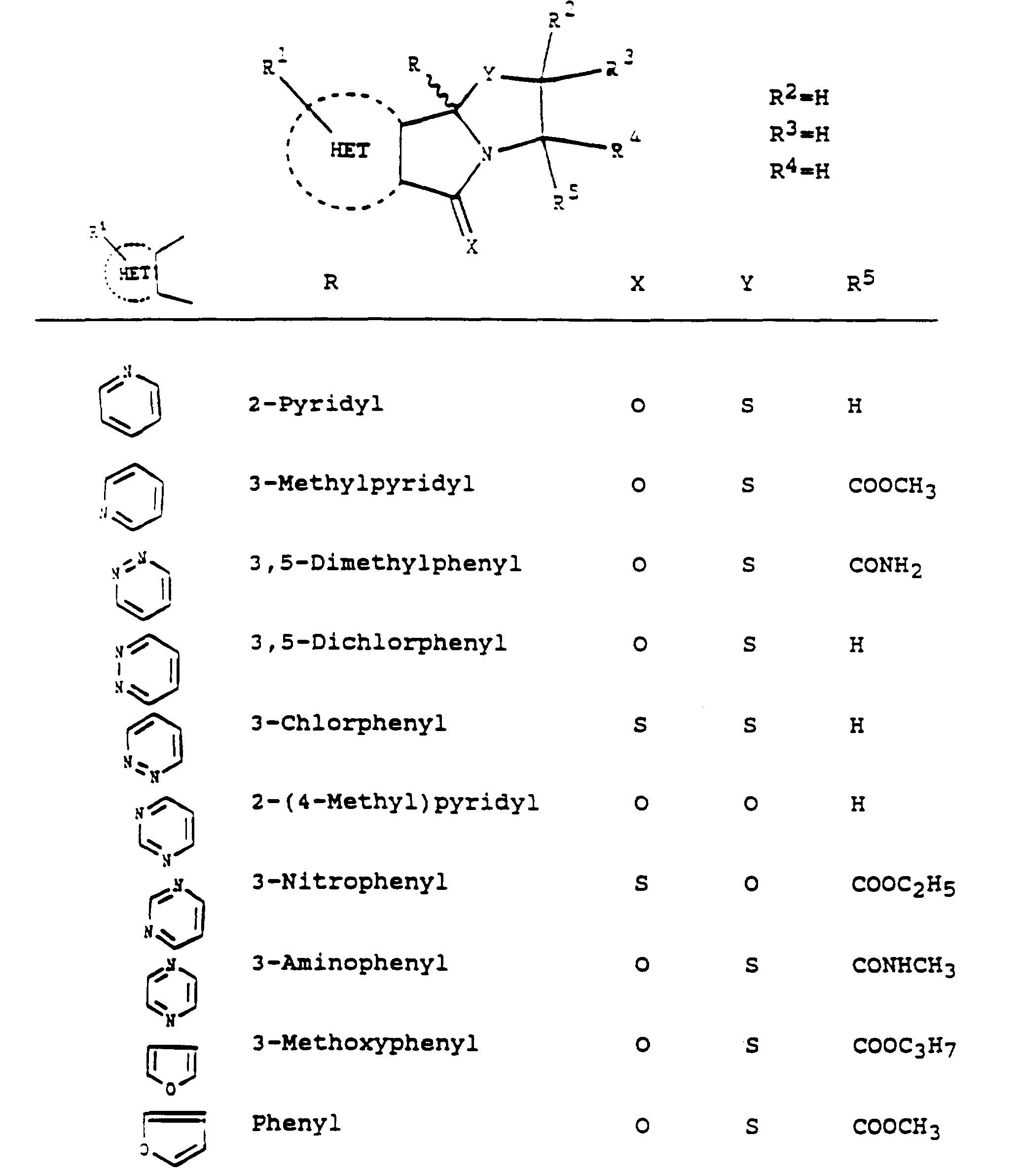

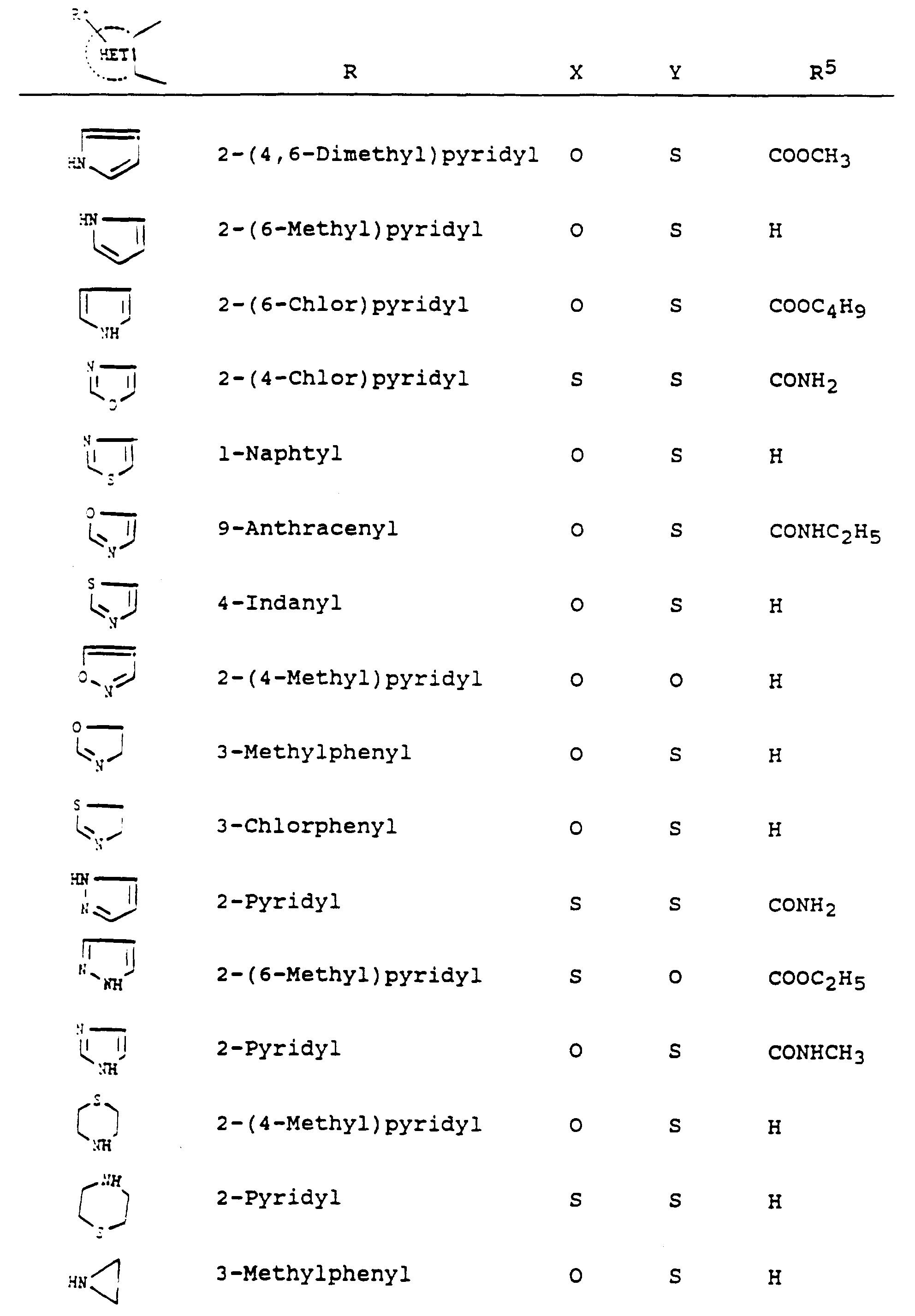

### Beispiel 1

### 3a-Phenyl-2,3a-dihydro-1H-3-thia-7,8a-diazacyclopenta[a]inden-8-one

a) 3-Benzoylpyridin-2-carbonsäure
   10 g (67 mmol) Chinolinsäureanhydrid wurden in 50 ml Benzol suspendiert und unter Eiskühlung portionsweise mit 17.9 g (134 mmol) AlCl₃ versetzt. Nach vollständiger Zugabe wurde 3 Stunden bei Raumtemperatur nachgerührt, vorsichtig mit Eis zersetzt und von dem vorhandenen Niederschlag abgesaugt. Das Rohprodukt wurde durch Säulenchromatographie an Kieselgel 60 mit Essigester/Dichlormethan 1/1 als Eluens gereinigt. Ausbeute 3.5 g,(23 % d.Th.)
b) 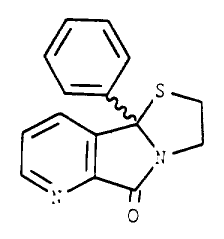
   1.2 g (5 mmol) 3-Benzoylpyridin-2-carbonsäure in 50 ml Toluol wurden mit 0.81 g (10 mmol) Cysteamin und einer kat. Menge p-Toluolsulfonsäure versetzt und 16 Stunden unter Rückfluß erhitzt. Nach dem Abkühlen wurde das Lösungsmittel im Vakuum entfernt und der Rückstand durch Säulenchromatographie an Kieselgel 60 mit Essigester/Isohexan 2/1 gereinigt. Die gewünschten Fraktionen wurden eingedampft und der Rückstand aus Ethanol umkristallisiert. Ausbeute 1.0 g (71 % d.Th.), Schmp. 178-179°C.

### Beispiel 2

### 3a-Phenyl-2,3a-dihydro-1H-3-thia-4,8a-diazacyclopenta[a]inden-8-one

a) 2-Benzoylpyridin-3-carbonsäure
   4.2 g Chinolinsäure-β-methylesterchlorid wurden in 40 ml Benzol suspendiert, bei 80°C portionsweise mit 8.4 g AlCl₃ versetzt und 4 Stunden bei 80°C gerührt. Dann wurde das Lösungsmittel im Vakuum entfernt, der Rückstand mit heißem Wasser extrahiert und der unlösliche Rückstand durch Säulenchromatographie an Kieselgel 60 mit Essigester/10% Methanol gereinigt. Ausbeute 1.42 g (30 % d.Th.).
b) 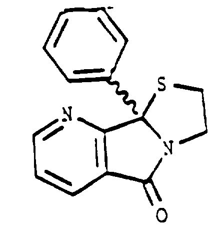
   1.3 g 2-Benzoylpyridin-3-carbonsäure wurden analog zu Bsp. 1 mit 0.85 g Cysteamin umgesetzt. Ausbeute 0.4 g (26 % d.Th.), Schmp. 172-176°C. [vgl. Monatshefte d. Chem. 31, 295 (1910)]

### Beispiel 3

### 3a-m-Tolyl-2,3a-dihydro-1H-3-thia-4,7,8a-triazacyclopenta[a]inden-8-one

a) 3-(m-Methylbenzoyl)-pyrazin-2-carbonsäure
   Das Grignardreagenz aus 8.5 g (50 mmol) 3-Bromtoluol und 1.2 g Magnesium in 50 ml Ether wurde bei 0 -10°C zu einer Lösung aus 7.5 g (50 mmol) Pyrazin-2,3-dicarbonsäureanhydrid in 100 ml THF getropft und die entstandene Lösung nach vollständiger Zugabe 4 h bei Raumtemperatur gerührt. Dann wurde mit 500 ml kalter, gesättigter NH₄Cl-Lsg. versetzt, mit Essigester extrahiert und die organische Phase getrocknet, eingedampft und wie unter Bsp. 1 a und 2 a gereinigt. Ausbeute 4 g (32 % d. Th.).
b) 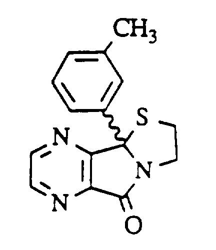
   1.5 g 3-(m-Methylbenzoyl)-pyrazin-2-carbonsäure wurden analog zu Bsp. 1 mit 1.15 g Cysteamin umgesetzt. Ausbeute 300 mg (17 % d. Th.), Schmp. 175°C.

### Beispiel 4

### 3a-Phenyl-2,3a-dihydro-1H-3-thia-4,7,8a-triazacyclopenta[a]inden-8-one

a) 3-Benzoylpyrazin-2-carbonsäure wurde analog zu Bsp. 3 a durch Verwendung von Phenylmagnesiumbromid in 56 % Ausbeute hergestellt.
b) 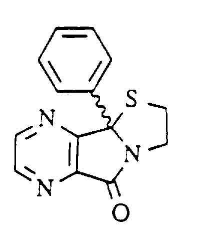
   1.15 g 3-Benzoylpyrazin-2-carbonsäure wurden analog zu Bsp. 1 mit 1.1 g Cysteamin in Xylol umgesetzt. Ausbeute 280 mg (20 % d. Th.), Schmp. 213 - 216°C.

### Beispiel 5

### 3a-(3,5-Dimethyl-phenyl)-2,3a-dihydro-1H-3-thia-4,7,8a-triaza-cyclopenta[a]inden-8-one

a) 3-(3,5-Dimethylbenzoyl)-pyrazin-2-carbonsäure wurde analog zu Bsp. 3 a durch Verwendung von 3,5-Dimethylphenylmagnesiumbromid in 32 % Ausbeute hergestellt.
b) 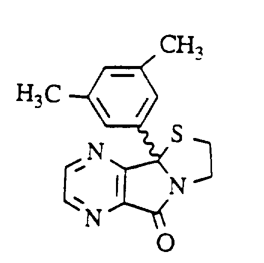
   2.6 g 3-(3,5-Dimethylbenzoyl)-pyrazin-2-carbonsäure wurden analog zu Bsp. 1 mit 2 g Cysteamin in Xylol umgesetzt. Ausbeute 805 mg (23 % d. Th.), Schmp. 162 - 165°C.

### Beispiel 6

### 3a-Phenyl-2,3a-dihydro-1H-3-thia-6,8a-diazacyclopenta[a]inden-8-one

a) 4-Benzoylpyridin-3-carbonsäure wurde analog zu Bsp. 1 a aus 3,4-Pyridindicarbonsäureanhydrid hergestellt und durch fraktionierende Kristallisation aus Ethanol von der 3-Benzoylpyridin-4-carbonsäure getrennt. Ausbeute 34 bzw. 27 % d. Th.
b) 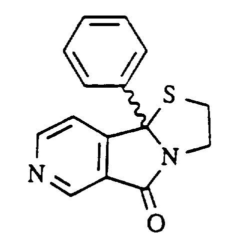
   1 g 4-Benzoylpyridin-3-carbonsäure wurden analog zu Bsp. 1 mit 680 mg Cysteamin umgesetzt. Ausbeute 440 mg (37 % d. Th.), Schmp. 120 - 121°C.

### Beispiel 7

### 3a-Phenyl-2,3a-dihydro-1H-3-thia-5,8a-diazacyclopenta[a]inden-8-one

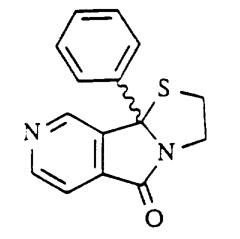

1.6 g der als Nebenprodukt bei Bsp. 6 a isolierten 3-Benzoylpyridin-4-carbonsäure wurden analog zu Bsp. 1 mit 1.08 g Cysteamin umgesetzt. Ausbeute 1.24 g (66 % d. Th.), Schmp. 228 - 229°C.

### Beispiel 8

### 3a-(4-Methyl-pyridin-2-yl)-2,3a-dihydro-1H-3-thia4,8a-diaza-cyclopenta[a]inden-8-one

a) 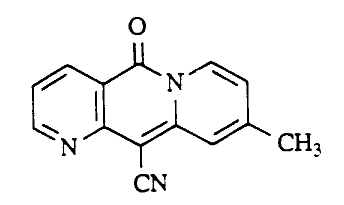
   Zu einer Suspension von 3.8 g (0.146 mol) NaH in 75 ml abs. Ether wurde innerhalb von 15 Min. eine Lösung aus 9.7 g (73 mmol) 2-Cyanomethyl-4-picolin in 25 ml Ether getropft und 1 h unter Rückfluß erhitzt. Dann wurden 13.5 g (73 mmol) 2-Chlornicotinsäuremethylester in 25 ml Ether zugegeben und weitere 3 h unter Rückfluß erhitzt. Nach dem Abkühlen wurden die entstandenen Kristalle abgesaugt und ohne weitere Reinigung in die nächste Reaktion eingesetzt.
b) 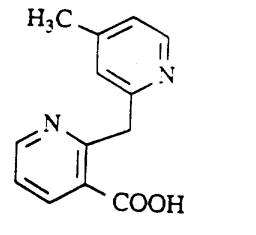
   Das Rohprodukt der letzten Reaktion in 150 ml 6 N HCl wurde 3 d im Autoklaven bei 130°C gerührt, die Kristalle abgesaugt und aus Ethanol umkristallisiert. Ausbeute 5.2 g (31 %, bezogen auf 2-Cyanomethyl-4-picolin), Schmp. 277 - 279°C.
c) 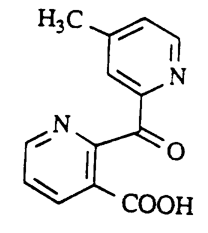
   1.6 g (7 mmol) 2-(4-Picolin-2-ylmethyl)nicotinsäure wurden mit 1.6 g KMnO₄ in 32 ml Wasser gelöst und 3 h auf 95°C erhitzt. Dann wurde der Braunstein abgetrennt, das Filtrat eingedampft und der Rückstand durch Säulenchromatographie an Kieselgel 60 mit CH₂Cl₂/EtOH 8/2 als Eluens gereinigt. Ausbeute 1.1 g (65 % d. Th.), Schmp. 210-212°C.
d) 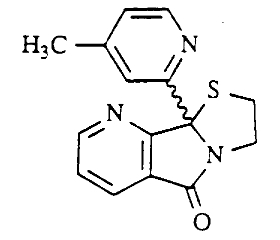
   0.7 g (3.1 mmol) 2-(4-Picolin-2-ylcarbonyl)-nicotinsäure wurden analog zu Bsp. 1 mit 0.5 g Cysteamin umgesetzt. Ausbeute 0.3 g (34 % d. Th.), Schmp. 129 - 130°C.

### Beispiel 9

### 3a-Pyridin-2-yl-2,3a-dihydro-1H-3-thia-4,8a-diaza-cyclopenta[a]inden-8-one

a) 2-(2-Pyridylcarbonyl)-nicotinsäure wurde analog zu Bsp. 8 ausgehend von 2-Cyanomethylpyridin hergestellt.
b) 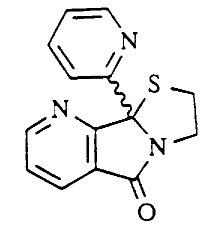
   1.4 g 2-(2-Pyridylcarbonyl)-nicotinsäure wurden analog zu Bsp. 1 mit 1 g Cysteamin umgesetzt. Ausbeute 670 mg (41 % d. Th.), Schmp. 149 -150°C.

### Beispiel 10

### 3a-(6-Methyl-pyridin-2-yl)-2,3a-dihydro-1H-3-thia-4,8a-diaza-cyclopenta[a]inden-8-one

a) 2-(2-Picolin-6-ylcarbonyl)-nicotinsäure wurde analog zu Bsp. 8 ausgehend von 6-Cyanomethyl-2-picolin hergestellt.
b) 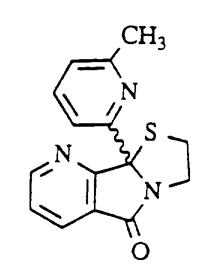
   1 g 2-(2-Picolin-6-ylcarbonyl)-nicotinsäure wurden analog zu Bsp. 1 mit 0.8 g Cysteamin umgesetzt. Ausbeute 655 mg (56 % d. Th.), Schmp. 134 - 135°C.

### Beispiel 11

### 3a-(4,6-Dimethyl-pyridin-2-yl)-2,3a-dihydro-1H-3-thia-4,8a-diaza-cyclopenta[a]inden-8-one

a) 2-(2,4-Lutidin-6-ylcarbonyl)-nicotinsäure wurde analog zu Bsp. 8 ausgehend von 6-Cyanomethyl-2,4-lutidin hergestellt.
b) 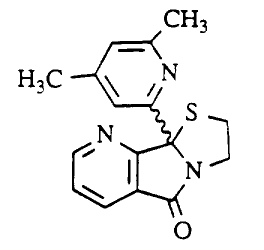
   870 mg 2-(2,4-Lutidin-6-ylcarbonyl)-nicotinsäure wurden analog zu Bsp. 1 mit 530 mg Cysteamin umgesetzt. Ausbeute 414 mg (41 % d. Th.), Öl.

### Beispiel 12

### 3a-(6-Methyl-pyridin-2-yl)-2,3a-dihydro-1H-3-oxa-4,8a-diaza-cyclopenta[a]inden-8-one

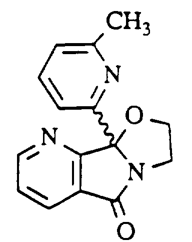

1.21 g 2-(2-Picolin-6-ylcarbonyl)-nicotinsäure wurden analog zu Bsp. 1 mit 610 mg Ethanolamin umgesetzt. Ausbeute 735 mg (55 % d. Th.), Schmp. 234 - 236°C.

### Beispiel 13

### 3a-m-Tolyl-2,3a-dihydro-1H-3-thia-4,8a-diazacyclopenta[a]inden-8-one

a) 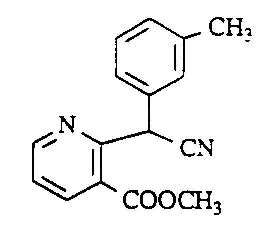
   Zu einer Suspension aus 7.92 g (0.33 Mol) NaH in 120 ml Ether wurden innerhalb von 15 Min. bei RT 19.6 g (0.15 Mol) m-Methylbenzylcyanid in 25 ml Ether getropft und 1 h unter Rückfluß erhitzt. Dann wurden bei RT 25.7 g 2-Chlornicotin-säuremethylester in 25 ml Ether zugetropft und weitere 2 h unter Rückfluß erhitzt. Nach dem Abkühlen wurde der entstandene Niederschlag abgesaugt, mit Wasser gewaschen und aus Isopropanol umkristallisiert, Ausbeute 31.2 g (78 % d. Th.), Schmp. 108 - 110°C.
b) 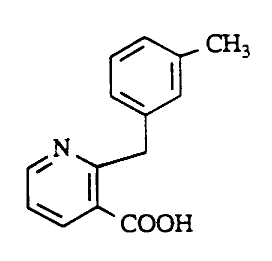
   Die Hydrolyse des Nitrils der letzten Reaktion erfolgte analog zu Bsp. 8 b. Ausbeute 43 %, Schmp. 73 - 77°C.
c) 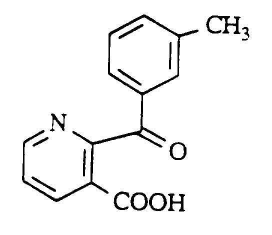
   Die Oxidation der 2-Benzylnicotinsäure der letzten Reaktion wurde analog zu Bsp. 8 c durchgeführt. Ausbeute 51 %, Schmp. 164-166°C.
d) 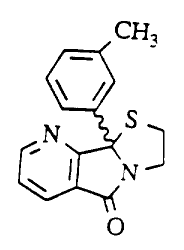
   960 mg 2-(m-Methylbenzoyl)-nicotinsäure wurden analog zu Bsp.1 mit 620 mg Cysteamin umgesetzt. Ausbeute 438 mg (39 % d. Th.), Schmp. 119 - 121°C.

### Beispiel 14

### 1-Hydrozymethyl-3a-m-tolyl-2,3a-dihydro-1H-3-thia4,8a-diaza-cyclopenta[a]inden-8-one

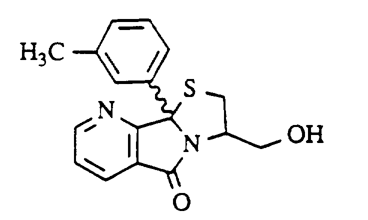

12 g (0.05 mol) 2-(3-Methylbenzoyl)nicotinsäure (vgl. Bsp. 13) wurden in Gegenwart von 10.7 g (0.1 mol) Cysteinol [vgl. Helv. Chim. Acta Vol. XLIV, Fasciculus III, 706 (1961), NO. 82; es wurde das Rohprodukt in die Reaktion eingesetzt] und 1.2 g p-Toluolsulfonsäure in 250 ml Toluol 4 h unter Rückfluß erhitzt. Nach dem Abkühlen wurde die Toluolphase mehrfach mit gesättigter NaHCO₃-Lsg. ausgeschüttelt, mit Wasser gewaschen, über Na₂SO₄ getrocknet und eingedampft. Der Rückstand wurde durch Säulenchromatographie an Kieselgel 60 mit Toluol/Aceton 5/2 als Eluens gereinigt. Ausbeute 4.2 g (27 % d. Th.).

### Beispiel 15

### 1-Methyl-3a-m-tolyl-3aH-3-thia-4,8a-diazacyclopenta[a]inden-8-one

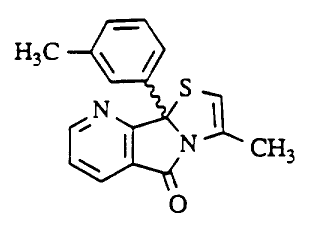

1.6 g (5.2 mmol) der Hydroxymethyl-Verbindung von Bsp. 14 in 30 ml abs. Pyridin wurden langsam mit 2 g (10.3 mmol) p-Toluolsulfonylchlorid versetzt und 24 h bei RT unter N₂ gerührt. Dann wurde das Lösungsmittel entfernt, der Rückstand in Essigester aufgenommen und je zweimal mit 2 N HCl und ges. NaHCO₃-Lsg. ausgeschüttelt. Die org. Phase wurde getrocknet, eingedampft und der Rückstand aus Isopropanol umkristallisiert.

Das Tosylat wurde danach in 70 ml Methanol gelöst, mit 0.3 ml 10 N NaOH sowie 0.21 g Imidazol versetzt und im Autoklaven 12 h auf 150°C erhitzt. Nach dem Abkühlen wurde das Lösungsmittel im Rotationsverdampfer abgezogen und der Rückstand durch PSC auf dem Chromatotron mit Toluol/Methanol 5/1 als Eluens gereinigt. Ausbeute 350 mg (23 % d. Th.).

### Beispiel 16

### Hemmung der Reversen Transkriptase

Das Screeningtestsystem beinhaltet die gereinigte RT aus HIV-1, die durch gentechnologische Methoden in E.coli exprimiert wurde, sowie die Komponennten des Initiationskomplexes und Site komplementären 18mer Oligonukleotid als Primer. Gemessen wurde der [3H]-Thymidin-5'-triphosphat-Einbau durch Auszählen im β-Counter. In der folgenden Tabelle wird für die untersuchten Verbindungen der IC₅₀-Wert angegeben. Dieser Wert entspricht derjenigen Konzentration der Testsubstanz, die eine Hemmung der RT-Aktivität um 50% bewirkt.

Ergebnisse:

| Substanz [Bsp.] | Hemmung der HIV-RT IC₅₀ [M] |
|---|---|
| 2 | 3,3 x 10⁻⁶ |
| 5 | 4,0 x 10⁻⁵ |
| 7 | 1,7 x 10⁻⁵ |
| 8 | 4,6 x 10⁻⁶ |
| 9 | 2,3 x 10⁻⁵ |
| 10 | 6,6 x 10⁻⁶ |
| 11 | 3,9 x 10⁻⁶ |
| 13 | 4,0 x 10⁻⁶ |

## Patentansprüche

1. Thiazolo-[2,3-a]pyrrol- und Oxazolo-[1,2-a]pyrrol-Derivate der Formel I 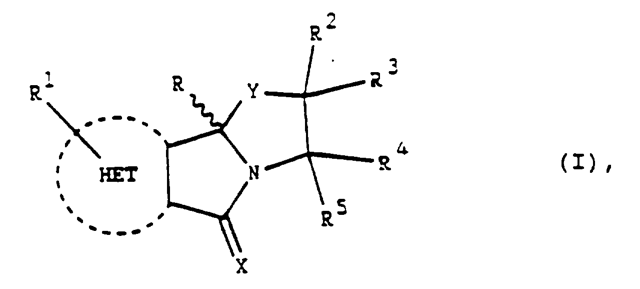 in der
HET einen aromatischen, teilhydrierten oder hydrierten hetero-cyclischen Ring mit 3-7 Ringatomen darstellt, von denen bis zu vier Heteroatome sein können, wobei die Heteroatome gleich oder verschieden sein können und Sauerstoff, Stickstoff oder Schwefel bedeuten, und die Heterocyclen gegebenenfalls an einem oder mehreren Stickstoffatomen ein Sauerstoffatom tragen können, wobei HET durch einen, zwei oder drei Reste R¹, die gleich oder verschieden sein können, substituiert sein kann,
Y ein Sauerstoff- oder Schwefelatom, bzw. die SO oder SO₂-Gruppe darstellt,
X ein Sauerstoff- oder Schwefelatom sein kann,
R einen geradkettigen oder verzweigten, gesättigten oder ungesättigten aliphatischen Rest mit 1-9 C-Atomen, der durch Phenyl substituiert sein kann,
oder einen Phenylring bedeutet, oder einen mono-, bi-oder tricyclischen carbocyclischen Ring mit 7-15 C-Atomen oder ein heterocyclisches mono-, bi- oder tricyclisches Ringsystem mit jeweils 5 oder 6 Ringatomen bedeutet und pro Ringsystem 1-4 bzw. 1-5 Heteroatome enthalten sein können und die Heteroatome Stickstoff, Schwefel oder Sauerstoff sind, wobei die Heteroatome Stickstoff und Schwefel gegebenenfalls durch Sauerstoffatome substituiert sein können,
und die vorgenannten Phenylringe, die mono-, bi-oder tricyclischen carbocyclischen Ringe oder das heterocyclische mono-, bi- oder tricyclische Ringsystem gegebenenfalls ein- oder mehrfach substituiert ist durch C₁-C₆-Alkyl, C₁-C₆-Alkoxy, C₁-C₆-Alkylmercapto, C₁-C₆-Alkylsulfinyl, C₁-C₆-Alkylsulfonyl, C₂-C₆-Alkenyl, C₂-C₆-Alkinyl, C₂-C₆-Alkenyloxy, C₂-C₆-Alkenylmercapto, C₂-C₆-Alkinyloxy, C₂-C₆-Alkinylmercapto, Amino, C₁-C₆-Alkylamino, Di-C₁-C₆-alkylamino, C₁-C₆-Alkylcarbonylamino, C₁-C₆-Alkylamino-carbonyl, C₁-C₆-Alkoxycarbonyl, Hydroxy, Benzyloxy, Phenylmercapto, Phenyloxy, Nitro, Cyano, Halogen, Trifluormethyl, Azido, Formylamino, Carboxy oder Phenyl,
R¹ ein Wasserstoffatom, einen geradkettigen oder verzweigten, gesättigten oder ungesättigten aliphatischen Rest mit 1-6 C-Atomen oder C₁-C₆-Alkoxy, C₁-C₆-Alkylmercapto, C₁-C₆-Alkylsulfinyl, C₁-C₆-Alkylsulfonyl, Amino, C₁-C₆-Alkylamino, Di-C₁-C₆-Alkylamino, Sulfonamido, C₁-C₆-Alkoxycarbonyl, Carboxy, Halogen, Hydroxy, Nitro, Cyano, Azido, Phenyl oder Benzyloxy bedeutet,
R Wasserstoff, C₁-C₆-Alkyl, C₁-C₆-Alkoxy, C₁-C₆-Alkylmercapto, Amino, C₁-C₆-Alkylamino, Di-C₁-C₆-Alkylamino, Halogen, Cyano, Hydroxy, Carboxy, C₁-C₆-Alkoxycarbonyl, Aminocarbonyl oder C₁-C₆-Alkylaminocarbonyl bedeutet,
R³, R⁴, R⁵ die gleiche Bedeutung wie R haben, wobei die Reste R, R³, R⁴ und R⁵ unabhängig voneinander gleich oder verschieden sein können, und R³ mit R⁴ gegebenenfalls eine Doppelbindung darstellen kann,
sowie deren Tautomere, Enantiomere, Diastereomere und physiologisch verträgliche Salze.

2. Verbindungen gemäß Anspruch 1, dadurch gekennzeichnet, daß HET einen aromatischen Fünf- oder Sechsring mit einem oder zwei Stickstoffatomen bedeutet.

3. Verbindungen gemäß Anspruch 1, dadurch gekennzeichnet, daß HET einen Fünf- oder Sechsring mit einem Sauerstoffatom oder einem Sauerstoff- und einem Stickstoffatom bedeutet.

4. Verbindungen gemäß Anspruch 1, dadurch gekennzeichnet, daß HET einen Fünf- oder Sechsring mit einem Schwefel- und einem Stickstoffatom bedeutet.

5. Verbindungen gemäß einem der Ansprüche 1-4, dadurch gekennzeichnet, daß R¹ Wasserstoff, C₁-C₇-Alkyl, C₂-C₇-Alkenyl, C₂-C₇-Alkinyl, C₁-C₇-Alkoxy, C₁-C₇-Alkylmercapto, C₁-C₇-Alkylamino, C₁-C₇-Alkoxycarbonyl, Amino, Halogen, Hydroxy, Cyano oder Azido bedeutet.

6. Verbindungen gemäß einem der Ansprüche 1-5, dadurch gekennzeichnet, daß R eine Phenylgruppe oder einen carbocyclischen Ring aus der Gruppe Naphtyl, Anthracenyl, Phenanthrenyl, Fluorenyl, Indenyl, Acenaphtylenyl, Norbornyl, Adamantyl, C₃-C₇-Cycloalkyl oder C₅-C₈-Cycloalkenyl bedeutet.

7. Verbindungen gemäß einem der Ansprüchen 1-5, dadurch gekennzeichnet, daß R einen heterocyclischen Ring aus der Gruppe Pyridin-, Pyrimidin-, Pyridazin-, Pyrazin-, Triazin-, Pyrrol-, Pyrazol-, Imidazol-, Triazol-, Thiazol-, Oxazol-, Isoxazol-, Oxadiazol-, Furazan-, Furan-, Thio-phen-, Indol-, Chinolin-, Isochinolin-, Cumaron-, Thio-naphthen-, Benzoxazol-, Benzthiazol-, Indazol-, Benzimid-azol-, Benztriazol-, Chromen-, Phthalazin-, Chinazolin-, Chinoxalin-, Methylendioxybenzol-, Carbazol-, Acridin-, Phenoxazin-, Phenothiazin-, Phenazin- oder Purinsystem, wobei diese Heterocyclen partiell oder vollständig hydriert sein können, bedeutet.

8. Verbindungen gemäß Anspruch 6 oder 7, dadurch gekennzeichnet, daß der Phenyl-, carbocyclische oder heterocyclische Ring substituiert ist durch C₁-C₇-Alkyl, C₂-C₇-Alkenyl- oder C₂-C₇-Alkinylgruppe oder eine Phenylgruppe bedeutet, wobei die Phenylgruppen ein- oder mehrfach substituiert sein können durch C₁-C₆-Alkyl, C₁-C₆-Alkoxy, C₁-C₆-Alkylmercapto, C₁-C₆-Alkylsulfinyl, C₁-C₆-Alkylsulfonyl, C₂-C₆-Alkenyl, C₂-C₆-Alkinyl, C₂-C₆-Alkenyloxy, C₂-C₆-Alkenylmercapto, C₂-C₆-Alkinyloxy, C₂-C₆-Alkinylmercapto, Amino, C₁-C₆-Alkylamino, Di-C₁-C₆-alkylamino, C₁- C₆-Alkylcarbonylamino, C₁-C₆-Alkylaminocarbonyl, C₁-C₆-Alkoxycarbonyl, Hydroxy, Benzyloxy, Phenylmercapto, Phenyloxy, Nitro, Cyano, Halogen, Trifluormethyl, Azido, Formylamino, Carboxy oder Phenyl.

9. Verbindungen gemäß einem der Ansprüche 1-8, dadurch gekennzeichnet, daß R-R⁵ unabhängig voneinander Wasserstoff oder C₁-C₆-Alkyl bedeuten, oder mindestens einer der Reste R-R⁵ C₁-C₆-Alkoxy, C₁-C₆-Alkylmercapto, Carboxy, C₁-C₆-Alkoxycarbonyl, Aminocarbonyl, C₁-C₆-Alkylaminocarbonyl, Halogen, Cyano oder Hydroxy bedeutet.

10. Verbindungen gemäß Anspruch 1 ausgewählt aus der Gruppe der folgenden Verbindungen:
3a-Phenyl-2,3a-dihydro-1H-3-thia-4,8a-diazacyclopenta[a]inden-8-one
3a-(4,6-Dimethyl-pyridin-2-yl)-2,3a-dihydro-1H-3-thia-4,8a-diaza-cyclopenta[a]inden-8-one
3a-m-Tolyl-2,3a-dihydro-1H-3-thia-4,8a-diazacyclopenta[a]inden-8-one

11. Verfahren zur Herstellung von Verbindungen der Formel I nach einem der Ansprüche 1-10, dadurch gekennzeichnet, daß man heterocyclische Ketocarbonsäuren der allgemeinen Formel II 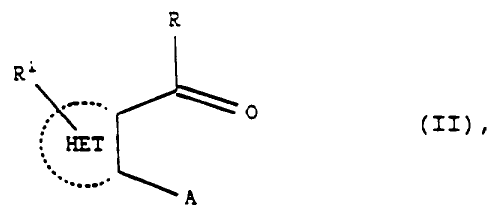 in der R und R¹ die oben angegebene Bedeutung haben und A gleich -COOH ist, mit substituiertem oder unsubstituiertem Cysteamin bzw. Ethanolamin der allgemeinen Formel III 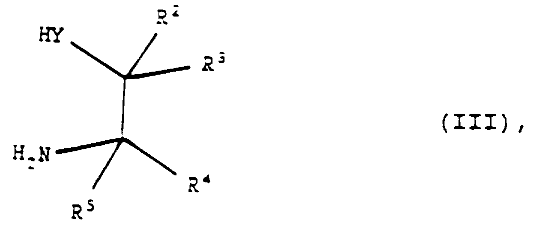 in der Y gleich Sauerstoff oder Schwefel ist und R, R³, R⁴ und R⁵ die oben angegebene Bedeutung haben, in einem geeigneten inerten Lösungsmittel bei Raumtemperatur bis Rückflußtemperatur evtl. in Gegenwart katalytischer Mengen Säure, z.B. p-Toluolsulfonsäure, umsetzt und gegebenenfalls anschließend Verbindungen der Formel I in andere Verbindungen der Formel I nachträglich umwandelt und anschließend chromatographisch bzw. durch Umkristallisation reinigt.

12. Arzneimittel enthaltend mindestens eine der Verbindungen nach den Ansprüchen 1-10, sowie pharmakologische Träger- oder Hilfsstoffe.

13. Verwendung von Verbindungen nach den Ansprüchen 1-10 zur Herstellung von Arzneimitteln mit antiviraler Wirkung.

## Claims

1. Thiazolo-[2,3-a]pyrrole and oxazole-[1,2-a]pyrrole derivatives of formula I 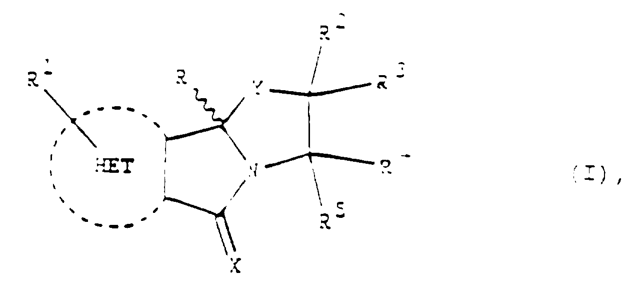 in which
HET represents an aromatic, partially hydrogenated or hydrogenated heterocyclic ring with 3-7 ring atoms of which up to four atoms can be heteroatoms in which the heteroatoms can be the same or different and denote oxygen, nitrogen or sulphur, and the heterocycles can, if desired, carry an oxygen atom on one or several nitrogen atoms and HET can be substituted by one, two or three residues R¹ which can be the same or different,
Y represents an oxygen or sulphur atom, or a SO or SO₂ group,
X can be an oxygen or sulphur atom,
R denotes a straight-chained or branched, saturated or unsaturated aliphatic residue with 1-9 C-atoms which can be substituted by phenyl or
denotes a phenyl ring or a mono, bi or tricyclic carbocyclic ring with 7-15 C-atoms or a heterocyclic mono, bi or tricyclic ring system each having five or six ring atoms and each ring system can contain 1-4 or 1-5 heteroatoms and the heteroatoms are nitrogen, sulphur or oxygen, in which the neteroatoms nitrogen and sulphur can, if desired, be substituted by oxygen atoms,
and the aforementioned phenyl rings, the mono, bi or tricyclic carbocyclic rings or the heterocyclic mono, bi or tricyclic ring system is substituted if desired, once or several times by C₁-C₆ alkyl, C₁-C₆ alkoxy, C₁-C₆ alkylmercapto, C₁-C₆ alkylsulfinyl, C₁-C₆ alkylsulfonyl, C₂-C₆ alkenyl, C₂-C₆ alkinyl, C₂-C₆ alkenyloxy, C₂-C₆ alkenylmercapto, C₂-C₆ alkinyloxy, C₂-C₆ alkinylmercapto, amino, C₁-C₆ alkylamino, di-C₁-C₆ alkylamino, C₁-C₆ alkylcarbonylamino, C₁-C₆ alkylaminocarbonyl, C₁-C₆ alkoxycarbonyl, hydroxy, benzyloxy, phenylmercapto, phenyloxy, nitro, cyano, halogen, trifluoromethyl, azido, formylamino, carboxy or phenyl,
R¹ denotes a hydrogen atom, a straight-chained or branched, saturated or unsaturated aliphatic residue with 1-6 C atoms or C₁-C₆ alkoxy, C₁-C₆ alkylmercapto, C₁-C₆ alkylsulfinyl, C₁-C₆ alkylsulfonyl, amino, C₁-C₆ alkylamino, di-C₁-C₆ alkylamino, sulfonamido, C₁-C₆ alkoxycarbonyl, carboxy, halogen, hydroxy, nitro, cyano, azido, phenyl or benzyloxy,
R denotes hydrogen, C₁-C₆ alkyl, C₁-C₆ alkoxy, C₁-C₆ alkylmercapto, amino, C₁-C₆ alkyl amino, di-C₁-C₆ alkylamino, halogen, cyanc, hydroxy, carboxy, C₁-C₆ alkoxycarbonyl, aminocarbonyl or C₁-C₆ alkylaminocarbonyl,
R³, R⁴, R⁵ have the same meaning as R and the residues R, R³, R⁴ and R⁵ can independently of one another be the same or different, and R³ with R⁴ can, if desired, represent a double bond,
as well as their tautomers, enantiomers, diastereomers and physiologically tolerated salts.

2. Compounds as claimed in claim 1, wherein HET denotes an aromatic five-membered or six-membered ring with one or two nitrogen atoms.

3. Compounds as claimed in claim 1, wherein HET denotes a five-membered or six-membered ring with an oxygen atom or with an oxygen and a nitrogen atom.

4. Compounds as claimed in claim 1, wherein HET denotes a five-membered or six-membered ring with a sulphur and nitrogen atom.

5. Compounds as claimed in one of the claims 1-4, wherein R¹ denotes hydrogen, C₁-C₇ alkyl, C₂-C₇ alkenyl, C₂-C₇ alkinyl, C₁-C₇ alkoxy, C₁-C₇ alkylmercapto, C₁-C₇ alkylamino, C₁-C₇ alkoxycarbonyl, amino, halogen, hydroxy, cyano or azido.

6. Compounds as claimed in one of the claims 1-5, wherein R denotes a phenyl group or a carbocyclic ring from the group naphthyl, anthracenyl, phenanthrenyl, fluorenyl, Indenyl, acenaphthylenyl, norbornyl, adamantyl, C₃-C₇ cycloalkyl or C₅-C₈ cycloalkenyl.

7. Compounds as claimed in one of the claims 1-5, wherein R denotes a heterocyclic ring from the group pyridine, pyrimidine, pyridazine, pyrazine, triazine, pyrrole, pyrazole, imidazole, triazole, thiazole, oxazole, isoxazole, oxadiazole, furazan, furan, thiophene, indole, quinoline, isoquinoline, coumarone, thionaphthene, benzoxazole, benzthiazole, indazole, benzimidazole, benztriazole, chromene, phthalazine, quinazoline, quinoxaline, methylene-dioxybenzol, carbazole, acridine, phenoxazine, phenothiazine, phenazine or purine system and these heterocycles can be partially or completely hydrogenated.

8. Compounds as claimed in claim 6 or 7, wherein the phenyl, carbocyclic or heterocyclic ring is substituted by a C₁-C₇ alkyl, C₂-C₇ alkenyl or C₂-C₇ alkinyl group or denotes a phenyl group, in which the phenyl groups can be substituted once or twice by C₁-C₆ alkyl, C₁-C₆ alkoxy, C₁-C₆ alkylmercapto, C₁-C₆ alkylsulfinyl, C₁-C₆ alkylsulfonyl, C₂-C₆ alkenyl, C₂-C₆ alkinyl, C₂-C₆ alkenyloxy, C₂-C₆ alkenylmercapto, C₂-C₆ alkinyloxy, C₂-C₆ alkinylmercapto, amino, C₁-C₆ alkylamino, di-C₁-C₆ alkylamino, C₁-C₆ alkylcarbonylamino, C₁-C₆ alkylaminocarbonyl, C₁-C₆ alkoxycarbonyl, hydroxy, benzyloxy, phenylmercapto, phenyloxy, nitro, cyano, halogen, trifluoromethyl, azido, formylamino, carboxy or phenyl.

9. Compounds as claimed in one of the claims 1-8, wherein R-R⁵ independently of each other denote hydrogen or C₁-C₆ alkyl or at least one of the residues R-R⁵ denotes C₁-C₆ alkoxy, C₁-C₆ alkylmercapto, carboxy, C₁-C₆ alkoxycarbonyl, aminocarbonyl, C₁-C₆ alkylaminocarbonyl, halogen, cyano or hydroxy.

10. Compounds as claimed in claim 1 selected from the group comprising the following compounds:
3a-phenyl-2,3a-dihydro-1H-3-thia-4,8a-diazacyclopenta[a]inden-8-one
3a-(4,6-dimethyl-pyridin-2-yl)-2,3a-dihydro-1H-3-thia-4,8a-diaza-cyclopenta[a]inden-8-one
3a-m-tolyl-2,3a-dihydro-1H-3-thia-4,8a-diazacyclopenta[a]inden-8-one

11. Process for the production of compounds of formula I as claimed in one of the claims 1-10, wherein ketocarboxylic acids of the general formula II 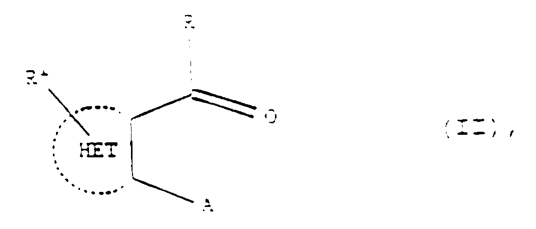 in which R and R¹ have the meanings stated above and A equals -COOH is reacted in a suitable inert solvent at room temperature to reflux temperature with substituted or unsubstituted cysteamine or ethanolamine of the general formula III 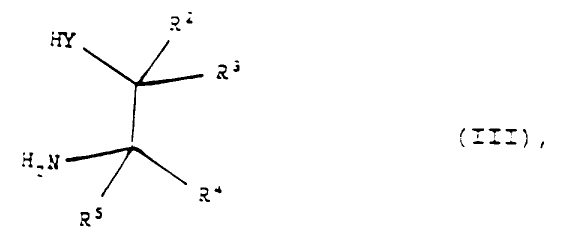 in which Y equals oxygen or sulphur and R, R³, R⁴ and R⁵ have the meanings stated above in the presence of catalytic amounts of acid e.g. p-toluenesulfonic acid and, if desired, compounds of formula I are subsequently converted into other compounds of formula I and subsequently purified by chromatography or recrystallization.

12. Pharmaceutical agents containing at least one of the compounds as claimed in claims 1-10 as well as pharmacological vehicles or auxiliary substances.

13. Use of compounds as claimed in claims 1-10 for producing pharmaceutical agents with antiviral action.

## Revendications

1. Dérivés de thiazolo-[2,3-a]pyrrol et d'oxazolo-[1,2-a]pyrrol de formule I 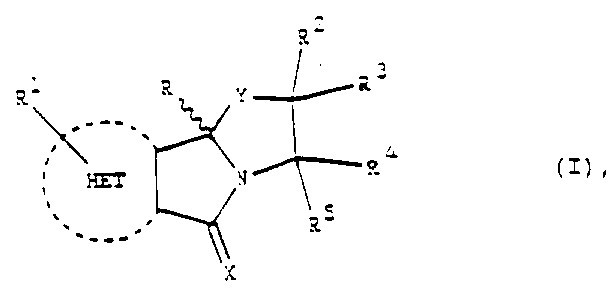 dans laquelle
HET représente un hétérocycle aromatique, partiellement ou totalement hydrogéné, comportant 3-7 maillons, dont jusqu'à 4 atomes peuvent être des hétéroatomes, les hétéroatomes pouvant être identiques ou différents et représenter l'oxygène, l'azote ou le soufre, et les hétérocycles pouvant éventuellement porter, sur un ou plusieurs atomes d'azote, un atome d'oxygène, HET pouvant porter, en tant que substituant, un, deux ou trois restes R¹, qui peuvent être identiques ou différents,
Y représente un atome d'oxygène ou de soufre, ou bien le groupe SO ou SO₂,
X peut représenter un atome d'oxygène ou de soufre,
R peut représenter un reste aliphatique saturé ou insaturé, à chaîne linéaire ou ramifiée, ayant 1-9 atomes de C, qui peut porter un substituant phényle, ou
un noyau phényle ou un composé carbocyclique mono-, bi- ou tricyclique ayant 7-15 atomes de C ou un système hétérocyclique mono-, bi- ou tricyclique ayant cinq ou six maillons par cycle et pouvant contenir, par système cyclique, 1-4 ou 1-5 hétéroatomes et les hétéroatomes pouvant représenter l'azote, le soufre ou l'oxygène, les hétéroatomes azote et soufre pouvant être substitués éventuellement par des atomes d'oxygène,
et le noyau phényle, le composé carbocyclique, mono-, bi- ou tricyclique, ou le système hétérocyclique mono-, bi- ou tricyclique mentionnés précédemment peuvent porter éventuellement un ou plusieurs substituants qui peuvent être un groupe alkyle en C₁-C₆, alcoxy en C₁-C₆, (alkyle en C₁-C₆)mercapto, (alkyle en C₁-C₆)sulfinyle, (alkyle en C₁-C₆)sulfonyle, alcényle en C₂-C₆, alcinyle en C₂-C₆, alcényloxy en C₂-C₆, (alcényloxy en C₂-C₆)mercapto, alcinyloxy en C₂-C₆, (alcinyle en C₂-C₆)mercapto, amino, alkylamino en C₁-C₆, di(alkyle en C₁-C₆)amino, (alkyle en C₁-C₆)carbonylamino, (alkyle en C₁-C₆)aminocarbonyle, (alcoxy en C₁-C₆)carbonyle, hydroxy, benzyloxy, phénylmercapto, phényloxy, nitro, cyano, halogéno, trifluorométhyle, azido, formylamino, carboxy ou phényle,
R¹ représente un atome d'hydrogène, un reste aliphatique saturé ou insaturé, à chaîne linéaire ou ramifiée, ayant 1-6 atomes de C, ou un reste alcoxy en C₁-C₆, (alkyle en C₁-C₆)mercapto, (alkyle en C₁-C₆)sulfinyle, (alkyle en C₁-C₆)sulfonyle, amino, (alkyle en C₁-C₆)amino, di(alkyle en C₁-C₆)amino, sulfonamido, (alcoxy en C₁-C₆)carbonyle, carboxy, halogéno, hydroxy, nitro, cyano, azido, phényle ou benzyloxy,
R représente un atome d'hydrogène, un groupe alkyle en C₁-C₆, alcoxy en C₁-C₆, (alkyle en C₁-C₆)mercapto, amino, (alkyle en C₁-C₆)amino, di(alkyle en C₁-C₆)amino, halogéno, cyano, hydroxy, carboxy, (alcoxy en C₁-C₆)carbonyle, aminocarbonyle ou (alkyle en C₁-C₆)aminocarbonyle,
R³, R⁴, R⁵ ont la même signification que R, les restes R, R³, R⁴ et R⁵ pouvant être indépendamment identiques ou différents, et R³ pouvant représenter, conjointement avec R⁴, éventuellement une double liaison,
ainsi que leurs tautomères, énantiomères, diastéréomères et leurs sels physiologiquement acceptables.

2. Composés selon la revendication 1, caractérisés par le fait que HET représente un cycle aromatique à cinq ou six chaînons ayant un ou deux atomes d'azote.

3. Composés selon la revendication 1, caractérisés par le fait que HET représente un cycle à cinq ou six chaînons ayant un atome d'oxygène ou un atome d'oxygène et un atome d'azote.

4. Composés selon la revendication 1, caractérisés par le fait que HET représente un cycle à cinq ou six chaînons ayant un atome de soufre et un atome d'azote.

5. Composés selon l'une quelconque des revendications 1-4, caractérisés par le fait que R¹ représente un atome d'hydrogène, un reste alkyle en C₁-C₇, alcényle en C₂-C₇, alcinyle en C₂-C₇, alcoxy en C₁-C₇, alkylmercapto en C₁-C₇, alkylamino en C₁-C₇, alcoxycarbonyle en C₁-C₇, amino, halogéno, hydroxy, cyano ou azido.

6. Composés selon l'une quelconque des revendications 1-5, caractérisés par le fait que R représente un groupe phényle ou un composé carbocyclique du groupe formé par les restes naphtyle, anthracényle, phénanthrényle, fluorényle, indényle, acénaphtényle, norbornyle, adamantyle ou un groupe cycloalkyle en C₃-C₇ ou cycloalcényle en C₅-C₈.

7. Composés selon l'une quelconque des revendications 1-5, caractérisés par le fait que R représente un hétérocycle appartenant au groupe formé par les systèmes pyridine, pyrimidine, pyridazine, pyrazine, triazine, pyrrol, pyrazole, imidazole, triazole, thiazole, oxazole, isoxazole, oxadiazole, furazane, furane, thiophène, indol, quinoléine, isoquinoléine, coumarone, thionaphtène, benzoxazole, benzothiazole, indazole, benzimidazole, benzotriazole, chromène, phtalazine, quinazoline, quinoxaline, méthylènedioxybenzène, carbazole, acridine, phénoxazine, phénothiazine, phénazine ou purine, ces hétérocycles pouvant être partiellement ou totalement hydrogénés.

8. Composés selon la revendication 6 ou 7, caractérisés par le fait que le noyau phényle, le composé carbocyclique ou hétérocyclique porte un substituant qui peut être un reste alkyle en C₁-C₇, alcényle en C₂-C₇ ou alcinyle en C₂-C₇ ou un groupe phényle, les groupes phényle pouvant porter un ou plusieurs substituants qui peuvent être les restes alkyle en C₁-C₆, alcoxy en C₁-C₆, (alkyle en C₁-C₆)mercapto, (alkyle en C₁-C₆)sulfinyle, (alkyle en C₁-C₆)sulfonyle, alcényle en C₂-C₆, alcinyle en C₂-C₆, alcényloxy en C₂-C₆, (alcényle en C₂-C₆)mercapto, alcinyloxy en C₂-C₆, (alcinyle en C₂-C₆)mercapto, amino, (alkyle en C₁-C₆)amino, di(alkyle en C₁-C₆)amino, (alkyle en C₁-C₆)carbonylamino, (alkyle en C₁-C₆)aminocarbonyle, (alcoxy en C₁-C₆)carbonyle, hydroxy, benzyloxy, phénylmercapto, phényloxy, nitro, cyano, halogéno, trifluorométhyle, azido, formylamino, carboxy ou phényle.

9. Composés selon l'une quelconque des revendications 1-8, caractérisés par le fait que R-R⁵ représentent indépendamment l'hydrogène ou un groupe alkyle en C₁-C₆, ou au moins un des restes R-R⁵ représente un groupe alcoxy en C₁-C₆, (alkyle en C₁-C₆)mercapto, carboxy, (alcoxy en C₁-C₆)carbonyle, aminocarbonyle, (alkyle en C₁-C₆)aminocarbonyle, halogéno, cyano ou hydroxy.

10. Composés selon la revendication 1, choisis dans le groupe des composés suivants :
3a-phényl-2,3a-dihydro-1H-3-thia-4,8a-diaza-cyclopenta[a]indène-8-one
3a-(4,6-diméthyl-pyridine-2-yl)-2,3a-dihydro-1H-3-thia-4,8a-diaza-cyclopenta[a]indène-8-one
3a-m-tolyl-2,3a-dihydro-1H-3-thia-4,8a-diaza-cyclopenta[a]indène-8-one

11. Procédé de préparation de composés de formule I selon l'une quelconque des revendications 1-10, caractérisé par le fait que l'on fait réagir un acide cétocarboxylique hétérocyclique de formule générale II 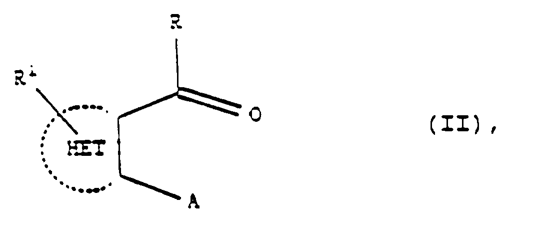 dans laquelle R et R¹ ont les significations mentionnées ci-dessus et A représente -COOH, avec une cystéamine ou éthanolamine substituée ou non substituée, de formule générale III 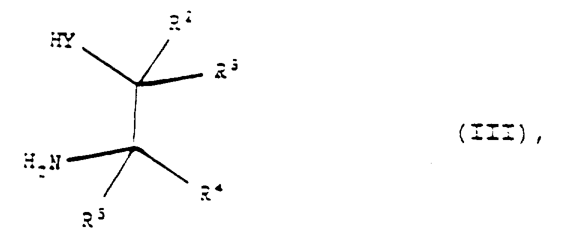 dans laquelle Y représente l'oxygène ou le soufre et R, R³, R⁴ et R⁵ ont les significations mentionnées ci-dessus, dans un solvant inerte approprié, à une température comprise entre la température ambiante et la température de reflux, éventuellement en présence de quantités catalytiques d'acide, par exemple l'acide p-toluènesulfonique, et éventuellement, on transforme ensuite les composés de formule I en d'autres composés de formule I, puis on les purifie par chromatographie ou par recristallisation.

12. Médicament contenant au moins un des composés selon les revendications 1-10, ainsi que des véhicules et adjuvants pharmaceutiquement acceptables.

13. Utilisation de composés selon les revendications 1-10 pour la préparation de médicaments ayant une action antivirale.
